Europäisches Patentamt

**European Patent Office**

Office européen des brevets

(19)

(11) Numéro de publication : **0 039 104 B1**

(12)

# FASCICULE DE BREVET EUROPÉEN

(45) Date de publication du fascicule du brevet :
11.05.83

(21) Numéro de dépôt : **81200405.9**

(22) Date de dépôt : **09.04.81**

(51) Int. Cl.³ : **C 12 N 11/00**

(54) **Procédé d'immobilisation de la bêta-galactosidase et produits ainsi obtenus.**

(30) Priorité : **25.04.80 BE 882984**

(43) Date de publication de la demande :
**04.11.81 Bulletin 81/44**

(45) Mention de la délivrance du brevet :
**11.05.83 Bulletin 83/19**

(84) Etats contractants désignés :
**DE FR GB NL**

(56) Documents cités :
**FR A 2 020 661**
**FR A 2 153 443**
**FR A 2 323 698**
**US A 4 229 536**
**POLYMER BULLETIN, vol. 1, no. 11, octobre 1979, Springer-Verlag, BERLIN (DE) C. G. BEDDOWS et al. : « The Use of Graft Copolymers as Enzyme Supports », pages 249-753**
**JOURNAL DAIRY SCIENCE, vol. 63, no. 2, février 1980, CHAMPAIGN-ILLINOIS (US) T. FINOC-CHIARO et al. : « Lactase Immobilized on Alumina », pages 215-222**
**AGRICULTURAL BIOLOGICAL CHEM., vol. 39, no. 2, 1975, TOKYO (JP) K. OHMIYA et al. : « Immobilization of beta-Galactosidase by Polyacrylamide in the Presence of Protective Agents », pages 491-498.**

(73) Titulaire : **Etat belge représenté par l'Exécutif de la Région Wallonne**
**11, Boulevard de l'Empereur**
**B-1000 Bruxelles (BE)**

(72) Inventeur : **Baijot, Bruno Faculté des Sc. Agronom. de l'Etat Dép. de Technol. agro-alimentaire et forestière groupe Ceteder B5800 Gembloux (BE)**
Inventeur : **Thonart, Philippe Faculté des Sc. Agronom. de l'Etat Dép. de Technol. agro-alimentaire et forestière groupe Ceteder B5800 Gembloux (BE)**
Inventeur : **Flambert, Franz Faculté dès Sc. Agronom. de l'Etat Dép. de Technol. agro-alimentaire et forestière groupe Ceteder B5800 Gembloux (BE)**
Inventeur : **Deroanne, Franz Faculté des Sc. Agronom. de l'Etat Dép. de Technol. agro-alimentaire et forestière groupe Ceteder B5800 Gembloux (BE)**

(74) Mandataire : **Blanchart, André**
**Unité de Recherches de Tubize ASBL 76/78, rue de Bruxelles**
**B-1360 Tubize (BE)**

Procédé d'immobilisation de la β-galactosidase et produits ainsi obtenus

La présente invention a pour objet un procédé d'immobilisation de la β-galactosidase sur un support insoluble possédant des groupes hydroxyles accessibles et les produits ainsi obtenus.

Les enzymes sont utilisées pour la catalyse des réactions chimiques très complexes présentant un grand intérêt, notamment pour l'industrie chimique et les industries agro-alimentaires.

Afin de pouvoir utiliser les enzymes dans des systèmes en continu, il s'est avéré nécessaire d'immobiliser les enzymes sur des supports insolubles. Cette fixation permet un rendement d'utilisation des enzymes beaucoup plus élevé et rend en plus les enzymes insolubles et plus stables.

La β-galactosidase ou lactase est une enzyme bien connue, reprise dans la nomenclature des enzymes sous la rubrique « Hydrolases » n° 3.2.1.23.

La β-galactosidase est utilisée pour l'hydrolyse du lactose en glucose et en galactose. La source de lactose peut être le lait, le lactosérum, ou divers jus lactosés obtenus comme sous-produits ou produits résiduaires du traitement du lait, du lactosérum ou d'autres sources lactosées.

L'hydrolyse du lactose se fait généralement au moyen d'une enzyme libre. Ces molécules d'enzyme ne peuvent généralement pas être séparées du milieu en fin de réaction et de ce fait, ne peuvent être utilisées qu'une seule fois, ce qui nuit à la pureté du produit obtenu et augmente le coût du traitement.

Par l'utilisation d'une enzyme immobilisée sur un support insoluble, le coût de cette hydrolyse peut être fortement diminué du fait notamment que l'enzyme peut être séparée du milieu et utilisée de nouveau.

De nombreuses méthodes ont été mises au point pour immobiliser la β-galactosidase. Une revue des problèmes se posant au sujet de la β-galactosidase, de ses procédés d'immobilisation et de l'hydrolyse du lactose, est donnée dans l'article de Shukla, T.P. publié dans C.R.C. Critical Reviews in Food Technology 1975, pp. 325-356.

Des différentes méthodes d'immobilisation de la β-galactosidase, celle par couplage de l'enzyme sur un support insoluble par liaisons covalentes constitue la méthode la plus étudiée du fait que l'insolubilisation de l'enzyme est la plus permanente. Toutefois, les préparations connues de la β-galactosidase immobilisée suivant cette méthode présentent différents inconvénients, par exemple, un nombre d'unités actives immobilisées faible et généralement des réactions de fixation complexes et longues.

En effet, la plupart des méthodes de couplage de l'enzyme par liaisons covalentes nécessitent plusieurs étapes souvent difficiles à mettre en œuvre faisant appel à l'utilisation de plusieurs réactifs. Les conditions de couplage sont telles, qu'elles risquent de modifier l'enzyme et donnent des rendements de couplage relativement faibles.

Un but de l'invention a été de réaliser l'immobilisation de la β-galactosidase sur un support insoluble, présentant des groupes hydroxyles accessibles, par un procédé simple, peu coûteux et rapide, permettant d'immobiliser un grand nombre d'unités actives sur le support.

Un autre but atteint par la présente invention est de permettre l'immobilisation de la β-galactosidase dans une zone de pH étendue.

Cette souplesse de préparation de la β-galactosidase immobilisée sur un support insoluble présentant des groupements hydroxyles accessibles est d'un grand intérêt industriel.

La présente invention a pour objet un procédé de préparation de β-galactosidase immobilisée sur un support insoluble présentant des groupes hydroxyles accessibles, activé par un diisocyanate aliphatique ou aromatique caractérisé en ce que la réaction du couplage de la β-galactosidase sur le support activé s'effectue en présence d'un ou de plusieurs monosaccharide(s), d'un ou de plusieurs disaccharide(s) ou de leurs mélanges.

Tout support, à condition qu'il porte des groupes hydroxyles accessibles, peut être utilisé. Le support doit être insoluble ou quasiment insoluble dans le milieu d'activation et de couplage. Les supports utilisés peuvent être de nature organique ou minérale.

Parmi les composés organiques pouvant être utilisés comme supports, citons les polysaccharides tels que la cellulose, ses dérivés et les polymères ou copolymères synthétiques.

Parmi les composés minéraux, citons l'utilisation de silice, de silicates et d'aluminosilicates, d'oxydes métalliques, etc... On peut utiliser, par exemple, le verre, le sable, la brique, la céramique, la pierre ponce, divers minéraux, les rebuts silicatés, etc... La matière organique ou minérale utilisée peut éventuellement être traitée en vue de lui conférer des groupements hydroxyles accessibles et/ou la rendre plus compatible à l'immobilisation de l'enzyme. Les composés organiques ou minéraux cités ci-avant ne constituent que des exemples de supports pouvant être utilisés suivant la présente invention et ne sont nullement limitatifs. Le choix du support tiendra compte à la fois de considérations physiques et mécaniques (porosité, résistance, etc...), économiques (disponibilité, prix, etc...) et opérationnelles (activité enzymatique, durée de vie, rendement, etc...).

Les supports utilisés peuvent se présenter sous des formes très diverses, par exemple de granules, de particules, de morceaux, de masses, de billes, de perles, de poudres, de flocons, de fils ou de fibres, de tissus ou de tricots, de films et de membranes. La densité apparente et l'état de division du support sont choisis notamment en fonction des conditions de préparation et d'utilisation de la β-galactosidase immobilisée.

Parmi les diisocyanates aliphatiques ou aromatiques utilisés pour l'activation du support, citons à titre d'exemple le diphénylméthane-4,4'-diisocyanate, le toluène-2,4-diisocyanate, le diisocyanate de xylylène et le diisocyanate d'hexaméthylène.

D'autres diisocyanates, tels que ceux habituellement utilisés pour l'immobilisation des enzymes ou pour les liaisons covalentes des enzymes entre-elles, peuvent être également utilisés.

Ces diisocyanates peuvent être utilisés seuls ou, éventuellement, en mélange.

La substance utilisée au moment du couplage de la β-galactosidase sur le support activé et qui fait l'objet de la présente invention comprend un ou plusieurs monosaccharide(s), un ou plusieurs disaccharide(s) ou leurs mélanges. A titre d'exemples de monosaccharides, citons les pentoses et les hexoses tels que l'arabinose, le glucose, le fructose et le mannose et de disaccharides : le lactose, le maltose et le saccharose. Conformément à l'invention, le lactose et le glucose sont les composés préférentiels.

Le procédé d'immobilisation de la β-galactosidase sur un support insoluble possédant des groupes hydroxyles accessibles comprend :

— d'une part, l'activation du support par un diisocyanate aliphatique ou aromatique, réaction bien connue ;

— d'autre part, la réaction de couplage de la β-galactosidase sur le support activé en présence d'un ou de plusieurs monosaccharide(s), d'un ou de plusieurs disaccharide(s), ou de leurs mélanges.

L'activation du support s'effectue par agitation de ce support dans une solution de diisocyanate choisi. Le diisocyanate est mis en solution, par exemple dans un solvant organique tel que l'acétone. Le solvant doit être inerte vis-à-vis du support et du diisocyanate et doit être choisi parmi les solvants les moins toxiques et les plus facilement éliminables par lavage afin qu'il ne laisse aucun résidu toxique dans le support activé.

Eventuellement, un catalyseur d'un type bien connu, tel qu'une amine tertiaire (la triéthylamine par exemple), un acide ou un métal peut être utilisé au cours de cette réaction d'activation du support.

En fin d'activation, le support est filtré et lavé.

La concentration en catalyseur de la solution de diisocyanate est choisie en fonction du degré de pureté du support et du pouvoir de réaction du diisocyanate. On augmentera la concentration pour compenser éventuellement la faible réactivité du support et/ou du diisocyanate utilisé(s). La concentration en diisocyanate dans la solution utilisée pour l'activation du support est choisie en fonction, notamment du nombre de groupements hydroxyles accessibles du support que l'on désire saturer.

La réaction de couplage consiste à agiter le support préalablement activé dans une solution tampon contenant l'enzyme, éventuellement un catalyseur et un ou plusieurs monosaccharide(s), un ou plusieurs disaccharide(s) ou leurs mélanges. L'enzyme ainsi immobilisée est filtrée, lavée dans une solution tampon et conservée dans cette même solution.

La solution tampon utilisée au cours de cette réaction de couplage est d'un type courant. Par exemple, on peut utiliser un tampon potassique. Le catalyseur peut éventuellement être celui utilisé au cours de la réaction d'activation du support. Le ou les mono- et/ou disaccharide(s) tels que le lactose, le glucose, le fructose, le mannose, le maltose, le saccharose ou l'arabinose doivent être utilisés au début de la réaction de couplage et être éventuellement présents dans la solution tampon contenant l'enzyme. Ces composés ajoutés à la solution de couplage sont présents à une concentration supérieure à 0,5 % et inférieure à la limite de solubilité dans le milieu. Les concentrations peuvent varier dans des limites relativement larges, suivant le type de support, de diisocyanate et le nombre de sites actifs du support activé devant réagir avec ceux de l'enzyme. L'optimum de la concentration en mono- et/ou disaccharides pourra être facilement fixée en déterminant l'activité enzymatique résiduelle atteinte par accroissement de la concentration. Par activité résiduelle, on entend l'activité de l'enzyme immobilisée par rapport à celle de la solution initiale d'enzymes libres. En général, l'optimum est atteint dans une zone de concentration comprise entre 0,5 % et 10 % et de préférence dans la zone de 1 % à 5 %.

L'effet surprenant de l'utilisation d'un mono- et/ou d'un disaccharide au cours de la réaction de couplage n'a pas encore trouvé d'explication scientifique.

En effet, en l'absence de mono- et/ou de disaccharide(s) ou d'utilisation de mono- et/ou de disaccharide(s) à la fin de la réaction de couplage, l'activité résiduelle reste faible.

La présente invention sera mieux comprise à l'aide des exemples non limitatifs ci-après :

## Exemple 1

Un gramme de billes de verre (Bio Glass-lOOO-BIORAD) est agité dans 20 ml d'acétone contenant 0,5 % de diphénylméthane-4,4'-diisocyanate et 0,5 % de triéthylamine. La réaction s'effectue à 4 °C et se prolonge pendant une heure. A la fin de cette réaction, le support est filtré et lavé avec de l'acétone et de l'eau.

Le support activé est alors agité dans 50 ml de tampon potassique 0,1 M de pH 6,6, contenant 0,2 % de triéthylamine, 2 % de lactose et respectivement 0,2 ml de lactozym 750 L (Novo-Danemark) pour le premier essai et 0,2 ml d'une enzyme provenant d'une bactérie EC pour le deuxième essai. La réaction s'effectue également à 4 °C, mais pendant une durée de 4 heures. A la fin de cette réaction, l'enzyme immobilisée est filtrée, lavée avec du tampon potassique 0,1 M de pH 6,6 et conservée dans ce même

**0 039 104**

tampon à 4 °C. Les mesures d'activité (test ONPG) ont montré que le rendement d'immobilisation est proche de 100 %.

Le tableau 1 donne l'activité résiduelle en % de la β-galactosidase de différentes provenances.

Tableau I

| Enzyme Provenance | Activité résiduelle de la β-galactosidase | | |
|---|---|---|---|
| | Lactose au début couplage | Lactose à la fin couplage | Pas de lactose |
| Levure (kluyvéro-myces lactis) | 71,4 | 39 | 39,3 |
| Bactérie E.C. | 79,0 | 43,8 | 42,1 |

Les résultats obtenus montrent nettement l'accroissement de l'activité résiduelle de la β-galactosidase immobilisée obtenue suivant le procédé de la présente invention par ajout de lactose au début du couplage.

### Exemple 2

Les conditions d'immobilisation sont celles de l'exemple 1, sauf que le support utilisé est de la pierre ponce. La pierre ponce, après avoir été broyée, est tamisée et la fraction utilisée est d'une granulométrie de 100 « mesh ». Vu la pureté moindre de ce support, il est nécessaire d'ajouter plus de réactif durant la réaction d'activation ; la concentration en triéthylamine est de 25 % et celle en diphénylméthane-4,4'-diisocyanate est de 2,5 %. Différents saccharides ont été ajoutés au début de la phase de couplage à une concentration de 2 %.

Les résultats obtenus sont donnés dans le tableau II. Le rendement de fixation des enzymes est voisin de 100 %.

Tableau II

| Sucre ajouté durant le couplage. | Activité résiduelle (%) |
|---|---|
| Pas de lactose | 30,2 |
| Lactose | 83,0 |
| Glucose | 83,0 |
| Mannose | 40,9 |
| Saccharose | 47,8 |
| Arabinose | 45,8 |

Les résultats montrent l'accroissement de l'activité résiduelle de la β-galactosidase par utilisation de divers mono- ou disaccharides. Le lactose et le glucose, qui sont suivant l'invention, les sucres préférentiels ajoutés durant le couplage, donnent les valeurs d'activité résiduelle les plus élevées.

### Exemple 3

Le tableau III montre l'influence de la concentration en lactose utilisé comme disaccharide durant le couplage. Des billes de verre activées au diphénylméthane-4,4' diisocyanate ont été agitées dans une solution tampon contenant de la β-galactosidase provenant d'une levure et du lactose à différentes

4

**0 039 104**

concentrations. Le mode opératoire est celui utilisé dans l'exemple 2.

Tableau III

| Concentration en lactose | Activité résiduelle. |
|---|---|
| 0 | 33 |
| 0,5 | 52 |
| 1 | 59 |
| 2 | 79 |
| 4 | 56 |

Les résultats montrent que, dans les conditions opérationnelles données ci-dessus, l'optimum se situe vers 2 %.

Exemple 4

Cet exemple est identique à l'exemple 1, sauf que le diisocyanate utilisé est le diisocyanate d'hexaméthylène avec utilisation de lactose ajouté au début de la réaction de couplage. Les résultats ont montré que 72 % des molécules d'enzyme initialement présentes sont immobilisées et que 74 % de ces molécules restent actives après l'immobilisation.

Exemple 5

Cet exemple montre l'avantage du procédé suivant l'invention de travailler dans une zone relativement large de pH pour la réaction de couplage de l'enzyme sur le support activé.

Un gramme de pierre ponce d'une granulométrie de 100 Mesh est agité dans 20 ml d'acétone contenant 2,5 % de diphénylméthane-4,4'-diisocyanate et 2,5 % de triéthylamine. Après une heure d'agitation, le support activé est filtré et lavé avec de l'acétone et de l'eau.

Le support activé est ensuite agité dans 50 ml de tampon Mc Ilvain de pH 3,6 contenat 15 mgr de β-galactosidase (5 μ/mg) extraite d'Aspergillus niger, 0,2 % de triéthylamine et 2 % de lactose. Un second essai a été effectué sans ajout de lactose. Après 4 heures de réaction à 4 °C, l'enzyme immobilisée est filtrée, lavée dans le tampon Mc Ilvain et conservée dans ce même tampon. Les résultats obtenus sont indiqués dans le tableau IV.

Tableau IV

| | Rendement | Activité résiduelle |
|---|---|---|
| Sans lactose | IOO | 42,9 |
| Avec lactose | 99,6 | 57,0 |

Les résultats montrent la possibilité de travailler à des pH faibles.

**Revendications**

1. Procédé d'immobilisation de la β-galactosidase sur un support insoluble présentant des groupes

5

hydroxyles accessibles, activé par un diisocyanate aliphatique ou aromatique, caractérisé en ce que la réaction de couplage de la β-galactosidase sur le support activé s'effectue en présence d'un ou de plusieurs monosaccharide(s), d'un ou de plusieurs disaccharide(s) ou de leurs mélanges.

2. Procédé suivant la revendication 1, caractérisé en ce que les monosaccharides et les disaccharides comportent des unités pentoses et/ou hexoses.

3. Procédé suivant la revendication 1 et 2, caractérisé en ce que les monosaccharides sont choisis parmi le glucose, le fructose, le mannose et l'arabinose et en ce que les disaccharides sont choisis parmi le lactose, le maltose et le saccharose.

4. Procédé suivant les revendications 1 à 3, caractérisé en ce que les mono- et/ou disaccharides sont choisis préférentiellement parmi le glucose et/ou le lactose.

5. Procédé suivant les revendications 1 à 4, caractérisé en ce que la réaction de couplage s'effectue dans une solution aqueuse contenant le ou les monosaccharide(s), le ou les disaccharide(s), à une concentration supérieure ou égale à 0,5 % et inférieure à leur limite de solubilité dans le milieu.

6. β-galactosidase immobilisée obtenue suivant les revendications 1 à 5.

## Claims

1. Process for immobilising β-galactosidase on an insoluble support having accessible hydroxyl groups activated by an aliphatic or aromatic diisocyanate, characterised in that the coupling reaction of the β-galactosidase on the activated support is effected in the presence of one or more monosaccharide(s), of one or more disaccharide(s) or mixtures thereof.

2. Process according to Claim 1, characterised in that the monosaccharides and the disaccharides comprise pentose and/or hexos units.

3. Process according to Claims 1 and 2, characterised in that the monosaccharides are chosen from glucose, frutose, mannose and arabinose, and that the disaccharides are chosen from lactose, maltose and saccharose.

4. Process according to Claims 1 to 3, characterised in that the mono and/or disaccharides are preferably chosen from glucose and/or lactose.

5. Process according to Claims 1 to 4, characterised in that the coupling reaction is effected in an aqueous solution containing the monosaccharide(s), the disaccharide(s), at a concentration above or equal to 0.5 % and below their limit of solubility in the medium.

6. Immobilised β-galactosidase obtained according to Claims 1 to 5.

## Ansprüche

1. Verfahren zur Immobilisierung von β-Galactosidase auf einem unlöslichen Träger, der verfügbare Hydroxylgruppen besitzt, aktiviert durch ein aliphatisches oder aromatisches Diisozyanat, dadurch gekennzeichnet, daß die Kupplungsreaktion der β-Galactosidase auf dem aktivierten Träger sich abspielt in Anwesenheit eines oder mehrerer Monosaccharide, eines oder mehrerer Disaccharide oder deren Mischungen.

2. Verfahren gemäß Anspruch 1 dadurch gekennzeichnet, daß die Monosaccharide und die Disaccharide Pentose- und/oder Hexose-Einheiten aufweisen.

3. Verfahren gemäß Anspruch 1 und 2, dadurch gekennzeichnet, daß die Monosaccharide ausgewählt sind unter Glucose, Fructose, Mannose und Arabinose und daß die Disaccharide ausgewählt sind unter Lactose, Maltose und Saccharose.

4. Verfahren gemäß Ansprüchen 1 bis 3 dadurch gekennzeichnet, daß die Mono- und/oder Disaccharide ausgewählt sind vorzugsweise unter Glucose und/oder Lactose.

5. Verfahren gemäß Ansprüchen 1 bis 4 dadurch gekennzeichnet, daß die Kupplungsreaktion sich in einer wässrigen Lösung abspielt, die das oder die Monosaccharide, das oder die Disaccharide in einer Konzentration oberhalb oder gleich 0,5 % und unterhalb ihrer Löslichkeitsgrenze in dem Milieu enthält.

6. Immobilisierte β-Galactosidase erhalten gemäß Ansprüchen 1 bis 5.